# EUROPEAN PATENT APPLICATION

(11) **EP 2 116 239 A1**
(43) Date of publication of application: **11.11.2009**
(21) Application number: 08290407.9
(22) Date of filing: 29.04.2008
(51) Int. Cl.: A61K 31/343, A61P 43/00

(54) **Method for managing the risks associated with an increase in serum creatinine during dronedarone treatment**

(71) Applicant: Sanofi-Aventis, 75013 Paris (FR)
(72) Inventor: Claudel, Sophie, 75013 Paris (FR); Gaudin, Christophe, 75013 Paris (FR)
(74) Representative: Gaslonde, Aude

(57) **Abstract**

A method of managing the risk of an increased morbidity and mortality in patients treated by an association of dronedarone and of at least a compound B selected from ACE inhibitors, angiotensin II receptor antagonists and potassium sparing diuretics which method comprises the following steps:
a) initially measuring serum creatinine levels, which provides a reference level,
b) monitoring serum creatinine levels at regular intervals during treatment of the patient by dronedarone and by the compound B,
c) if the serum creatinine levels increases above the reference level, determining if the increase is due to dronedarone, to compound B or to another cause,
d) if the increase in the serum creatinine level above the reference level is due to dronedarone, then the treatment with dronedarone and compound B can be pursued; if the increase in the serum creatinine level above the reference level is due to compound B, then the treatment with compound B may be interrupted; if the increase in the serum creatinine level above the reference level is due to another cause than administration of dronedarone or of compound B, then appropriate treatment of such another cause shall be undertaken,
e) repeating steps b), c) and d) for the duration of the treatment with dronedarone and compound B.

## Description

The instant invention relates to a method of managing the risk due to an observation of serum creatinine increase leading to inappropriate management of patients with CHF (Congestive Heart Failure). The instant invention more specifically relates to a method of managing the risk due to an observation of serum creatinine increase in patients treated by dronedarone and by ACE inhibitors or angiotensin II antagonists or potassium sparing diuretics.

2-n-butyl-3-[4-(3-di-n-butylaminopropoxy)benzoyl]-5-methylsulfonamido-benzofuran, or dronedarone, and its pharmaceutically acceptable salts are described in the European patent EP 0 471 609 B1.

Dronedarone is an antiarrhythmic agent effective in the reduction of cardiovascular hospitalization and death in patients with atrial fibrillation or atrial flutter or with a history of atrial fibrillation or atrial flutter. By the oral route, dronedarone is administered at daily doses up to 800 mg, in one or several intakes.

Creatinine is an end-product of muscle metabolism, which is freely filtered at the glomerulus and not metabolized in the kidney. It is secreted by the proximal tubules by both the anionic and the cationic secretory pathways. Usually an increase in creatinine level is considered as a marker of decreased glomerular filtration and as a sign of renal impairment (Journal of Internal medicine, 1999, 246, 247-252).

During clinical trials with dronedarone, it has been observed that administration of this active principle is associated with a slight increase in the serum creatinine levels of the patient. This increase occurred early after treatment initiation (within two days). Creatinine levels remained stable during treatment and returned to baseline within three days after treatment discontinuation.

It has however been demonstrated that such an increase in the creatinine levels is not linked to renal impairment, but is due to decreased secretion of this substance at the kidney tubular level. In fact, in a specific study in healthy subjects, this increase was shown to be related to inhibition of creatinine secretion at the tubular level, with no effect on glomerular filtration or on renal blood flow. Hence dronedarone can not be considered as a nephrotoxic agent. This decrease in the tubular secretion of creatinine is also reported with other drugs such as cimetidine, trimethoprim or even amiodarone (Br. J. Clin. Pharmac., 1993, 36, 125-127).

This effect of dronedarone on the creatinine levels becomes of particular relevance when this agent is co-prescribed to patients receiving another therapy that could interact with kidney function. This is especially the case for the treatment of patients with cardiovascular disease such as patients with heart failure, coronary disease and other cardiovascular risk factors receiving ACE inhibitors and/or angiotensin II receptor antagonists and/or spironolactone or other potassium sparing diuretics. Indeed, such categories of active ingredients are known for their adverse effects on the renal function. In patients treated both by dronedarone and by ACE inhibitors and/or angiotensin II receptor antagonists and/or potassium sparing diuretics, an increase in creatinine levels might be misinterpreted by the physician as a sign of nephrotoxicity of ACE inhibitors or angiotensin II receptor antagonists or potassium sparing diuretics, leading to inappropriate discontinuation of these agents. These treatments have been shown to prevent mortality in patients with heart failure (Pfeffer MA et al., Effect of captopril on mortality and morbidity in patients with left ventricular dysfunction after myocardial infarction. Results of the survival and ventricular enlargement trial. The SAVE Investigators, N. Engl. J. Med. 1992 Sep 3;327(10):669-77; Pitt B et al., The effect of spironolactone on morbidity and mortality in patients with severe heart failure. Randomized Aldactone Evaluation Study Investigators, N. Engl. J. Med. 1999;341:709-717; Pitt B et al., Eplerenone, a selective aldosterone blocker, in patients with left ventricular dysfunction after myocardial infarction., N. Engl. J. Med. 2003;348:1309-1321) and in patients with coronary disease (Indications for ACE inhibitors in the early treatment of acute myocardial infarction: systematic overview of individual data from 100,000 patients in randomized trials. ACE Inhibitor Myocardial Infarction Collaborative Group. Circulation 1998;97:2202-2212; Swedberg K et al., Effects of the early administration of enalapril on mortality in patients with acute myocardial infarction. Results of the Cooperative New Scandinavian Enalapril Survival Study II (CONSENSUS II)., N. Engl. J. Med. 1992;327:678-684; Dickstein K et al., Effects of losartan and captopril on mortality and morbidity in high-risk patients after acute myocardial infarction: the OPTIMAAL randomised trial. Optimal Trial in Myocardial Infarction with Angiotensin II Antagonist Losartan, Lancet 2002;360:752-760) or to prevent events, including cardiovascular mortality, in patients at cardiovascular risk (Yusuf S et al., Effects of an angiotensin-converting-enzyme inhibitor, ramipril, on cardiovascular events in high-risk patients. The Heart Outcomes Prevention Evaluation Study Investigators., N. Engl. J. Med. 2000 Jan 20;342(3):145-53). Inappropriate interruption of the treatment with ACE inhibitors or angiotensin II receptor antagonists or potassium sparing diuretics therefore exposes these patients to an increased cardiovascular morbidity and mortality.

The Applicant has now found a method for managing such a risk. The method according to the invention enables to decrease the risk of an inappropriate interruption of ACE inhibitors, of angiotensin II receptor antagonists treatment or of potassium sparing diuretics, which consequently enables to decrease the risk of morbidity and mortality of the patient.

The instant invention therefore relates to a method of managing the risk of an increased morbidity and mortality in patients treated by an association of dronedarone and of at least a compound B selected from ACE inhibitors, angiotensin II receptor antagonists and potassium sparing diuretics. Said method is performed by performing the following steps:
a) initially measuring serum creatinine levels, such as within one week after initiation of treatment with dronedarone, which provides a reference level,
b) monitoring serum creatinine levels at regular intervals during treatment of the patient by the compound B, (as recommended in the labelling of compound B),
c) if the serum creatinine levels increases above the reference level, clinical judgement should be made to determine if the increase is due to dronedarone, to compound B or to another cause,
d) to help clinical judgment and evaluate if the increase is due to dronedarone, dronedarone can be temporarily interrupted. The return to baseline within 5 days will be highly suggestive of dronedarone responsibility, as demonstrated in another clinical trial. If despite discontinuation, creatinine levels do not decrease, another cause including other treatments should be considered.
   - if, by this mean, the increase is thought to be due to dronedarone, concomitant treatment with B can be pursued with regular monitoring of creatinine levels,
   - if the increase in the serum creatinine level above the reference level is thought to be due to compound B, then labelling instructions of compound B should be followed and alternative treatment could be considered,
   - if the increase in the serum creatinine level above the reference level is due to another cause than administration of dronedarone or of compound B, then appropriate treatment of such cause shall be undertaken,
e) repeating steps b), c) and d) for the duration of the treatment with dronedarone and compound B.

The instant invention further relates to a method of managing the risk of an increased morbidity and mortality in patients treated by an association of dronedarone and of at least a compound B selected from ACE inhibitors, angiotensin II receptor antagonists and potassium sparing diuretics, which method comprises the following steps:
a) initially measuring serum creatinine levels, which provides a reference level,
b) monitoring serum creatinine levels at regular intervals during treatment of the patient by dronedarone and by the compound B,
c) if the serum creatinine levels increases above the reference level, determining if the increase is due to dronedarone, to compound B or to another cause,
d) if the increase in the serum creatinine level above the reference level is due to dronedarone, then the treatment with dronedarone and compound B can be pursued; if the increase in the serum creatinine level above the reference level is due to compound B, then the treatment with compound B may be interrupted; if the increase in the serum creatinine level above the reference level is due to another cause than administration of dronedarone or of compound B, then appropriate treatment of such another cause shall be undertaken,
e) repeating steps b), c) and d) for the duration of the treatment with dronedarone and compound B.

The instant invention further relates to a method of treatment of patients with cardiac arrhythmia comprising the administration of dronedarone to patients treated, in addition, by at least a compound B selected from ACE inhibitors, angiotensin II receptor antagonists and potassium sparing diuretics, which method comprises the following steps:
a) initially measuring serum creatinine levels, which provides a reference level,
b) monitoring serum creatinine levels at regular intervals during treatment of the patient by dronedarone and by the compound B,
c) if the serum creatinine levels increases above the reference level, determining if the increase is due to dronedarone, to compound B or to another cause,
d) if the increase in the serum creatinine level above the reference level is due to dronedarone, then the treatment with dronedarone and compound B can be pursued; if the increase in the serum creatinine level above the reference level is due to compound B, then the treatment with compound B may be interrupted; if the increase in the serum creatinine level above the reference level is due to another cause than administration of dronedarone or of compound B, then appropriate treatment of such another cause shall be undertaken,
e) repeating steps b), c) and d) for the duration of the treatment with dronedarone and compound B.

The instant method of treatment can be more specifically directed to the treatment of patients with atrial fibrillation or atrial flutter.
Examples of ACE inhibitors may be captopril, enalapril, perindopril, quinapril, lisinopril, ramipril, etc...
Examples of angiotensin II receptor antagonists may be losartan, valsartan, candesartan, telmisartan, irbesartan, etc...
Examples of potassium sparing diuretics may be spironolactone, eplerenone, etc...

Such compounds are usually prescribed for prevention and treatment of various pathologies of the cardiac function, such as cardiovascular disorders, congestive heart failure, left ventricular dysfunction or hypertension.

As regards to dronedarone, the daily dose by the oral route is up to 800 mg, in one or several intakes.
In specific cases, higher or lower dosages may be appropriate; these dosages are comprised within the scope of the present invention. According to usual practice, the dosage suitable to each patient is determined by the physician according to the administration route, the weight and response of the patient.
The appropriate unitary dosage forms for dronedarone comprise the oral forms, such as tablets, hard or soft gelatin capsules, powders, granules and oral solutions or suspensions, as well as the sublingual, buccal, intratracheal, intraocular, intranasal forms, the forms adapted to inhalation, topical, transdermal, sub-cutaneous, intramuscular or intra-venous delivery, the rectal forms and the implants. For the topical application, dronedarone may be used as creams, gels, ointments or lotions.

Concerning step a) of the methods of the invention, as mentioned before, in most patients with atrial fibrillation or atrial flutter the maximal increase in creatinine levels, compared to the level before administration of dronedarone, was smaller than 30%, with a mean increase ranging from 10 to 15% or more. Then during the treatment with dronedarone, mean creatinine levels remain stable.

In step a) of the methods according to the invention, the serum creatinine levels may in particular be measured one week after the start of dronedarone administration to the patient.
By "initially" in step a), one may for instance mean "one week after initiation of treatment with dronedarone", being understood that a few days less or more are also encompassed within the scope of the step a), so that the reference level for serum creatinine may be measured at 3 to 11 days, for example, after start of dronedarone administration to the patient.

In step b) of the methods according to the invention, the monitoring of the serum creatinine levels at regular intervals means that the serum creatinine shall be measured over the total duration of the treatment with dronedarone and compound B, based on a schedule depending on the specific pathological state of the patient and on the prescription labelling for the compound B. Such monitoring may for example be performed every 2 months in patients with renal impairment.

Step c) of the methods according to the instant invention targets to identify whether dronedarone or compound B may be the cause of the increase in the creatinine level above the reference level, or if it may be due to another cause, i.e. an outside cause than the active principles administered to the patient. In fact, various pathological states can be responsible for transient or permanent creatinine elevation, for example diabetes or impaired cardiac function, deshydration, hypovolemia, renal impairment, drug toxicity etc... Thus, a deterioration of the pathological state of the patient under the treatment of dronedarone and of compound B may involve serum creatinine elevation.

Step c) may be achieved by temporarily interrupting treatment with dronedarone, while maintaining treatment with compound B. Indeed, compound B should not be primarily interrupted unless there is a specific reason, especially in CHF patients.
The temporary interruption of dronedarone treatment may for example range from 1 to 2 weeks. Then the serum creatinine level shall be measured. If it returns to the reference level, then dronedarone treatment shall be reinstated. Indeed, this would mean that the increase in creatinine was due to dronedarone itself, and not related to any other concomitant disease including other drug toxicity.
On the other hand, if the creatinine level does not return to the reference level after the dronedarone treatment interruption, then it would imply that dronedarone is not the cause of the further creatinine increase. In such a case it shall be determined whether the increase is due to compound B, in which case treatment with compound B may be interrupted, or at least closely monitored to avoid renal impairment, or if the increase is due to another cause, as described above, in which case appropriate treatment of such other cause shall be undertaken. Appropriate treatment shall be appreciated by the physician depending on the patient's clinical condition and associated pathologies.

The instant invention is illustrated by the clinical data below.

### Example 1

As a preliminary comment, it has been observed and later demonstrated in a clinical trial in 627 patients with a recent severe episode of Congestive Heart failure (ANDROMEDA) that among all prognostic factors, the most important risk factor for death in patients receiving the dronedarone concomitantly, was the absence of treatment with ACE inhibitors or angiotensin II receptor antagonists (table 1).

**Table 1: Adjusted relative risk of time from randomization to death by prognostic factors up to 16 January 2003 - all randomized and treated patients with a recent severe episode of CHF (EFC4966/ANDROMEDA)**

| **Prognostic factor** | **Risk** | **Adjusted relative risk** (a) | | |
|---|---|---|---|---|
| | | **Relative risk** | **95% CI** (b) | **P-value** (c) |
| ACE inhibitor or angiotensin II receptor No antagonist | Intake / intake | 0.21 | [0.097; 0.432] | 0.00003 |

| | | | | |
|---|---|---|---|---|
| (a) Determined from Cox regression model. (b) Confidence Interval. (c) Statistical significance. | | | | |

### Example 2

Despite the equal distribution of treatment with ACE inhibitors/All receptor antagonists at baseline, concomitant treatment with these drugs became unbalanced during the course of the ANDROMEDA study, as more patients in the dronedarone 400 mg BID group discontinued these treatments and fewer started them (table 2).

**Table 2- Number (%) of patients according to distribution of ACE inhibitor/All receptor antagonist intake - all randomized and treated patients with a recent severe episode of CHF (EFC4966/ANDROMEDA)**

| | **Placebo (N=317)** | **Dronedarone 400 mg BID (N=310)** |
|---|---|---|
| Patients with ACE inhibitors or All receptor antagonists at baseline | 267 (84.2%) | 274 (88.4%) |
| Patients with ACE inhibitors or All receptor antagonists at baseline who did not interrupt these treatments | 254 (80.1%) | 237 (76.5%) |
| Patients who started ACE inhibitors or All receptor antagonists after baseline who did not interrupt these treatments | 27 (8.5%) | 12 (3.9%) |
| Patients who discontinued treatment with ACE inhibitors or All receptor antagonists | 18 (5.7%) | 41 (13.2%) |
| Patients who were never treated with ACE inhibitors or All receptor antagonists | 18 (5.7%) | 20 (6.5%) |

| | | |
|---|---|---|
| Among patients who interrupted, or did not start treatment with ACE inhibitors/All receptor antagonists (hereinafter "ACE/All"), more patients in the dronedarone 400 mg BID group died, as opposed to patients who never interrupted ACE inhibitors/All receptor antagonists (3.6% and 4%, respectively in placebo and dronedarone 400 mg BID groups) as shown in table 3. | | |

**Table 3 -Number (%) of patients who died according to the intake of ACE inhibitors/All receptors antagonists up to 16 January 2003 - all randomized and treated patients with a recent severe episode of CHF (EFC4966/ANDROMEDA)**

| | **Placebo (N=317)** | **Dronedarone 400 mg BID (N=310)** |
|---|---|---|
| Never interrupted concomitant ACE inhibitors or All receptor antagonists | 10 / 281 (3.6 %) | 10 / 249 (4 %) |
| Never took or interrupted concomitant ACE inhibitors or All receptor antagonists | 2 / 36 (5.6 %) | 15 / 61 (24.6 %) |

### Example 3

Table 4 shows how an increase in creatinine level induces the physicians to interrupt the treatment with ACE/All.

**Table 4: Number (%) of patients who stopped ACE/All and who had increased serum creatinine during the period of treatment - All randomized and treated patients with a recent severe episode of CHF (EFC4966/ANDROMEDA)**

| **Adversed effects** | **Placebo n=317** | | **Dronedarone 400 mg BID n=310** | |
|---|---|---|---|---|
| Blood creatinine increased | 0 / 18 | (0.0%) | 13 / 41 | (31.7%) |
| Renal impairment | 0 / 18 | (0.0%) | 1 / 41 | (2.4%) |

In the dronedarone 400 mg BID group, 14 of 41 patients who discontinued ACE/All experienced "blood creatinine increased" or "renal impairment" adverse events, versus none among the 18 patients taking placebo in this category. This observation supports the hypothesis that an increase in serum creatinine observed in patients taking dronedarone may have led investigators to discontinue ACE/All treatment (table 4).

### Example 4

In the ATHENA trial, the above mentioned instructions to appropriately handle plasma creatinine increase and ACE inhibitors/All receptor antagonists have been shown to be effective for the prevention of cardiovascular mortality and cardiovascular morbidity (hospitalizations for cardiovascular reasons), including in patients with different status of renal function, i.e different values of creatinine clearance at baseline (figure 1 and figure 2).

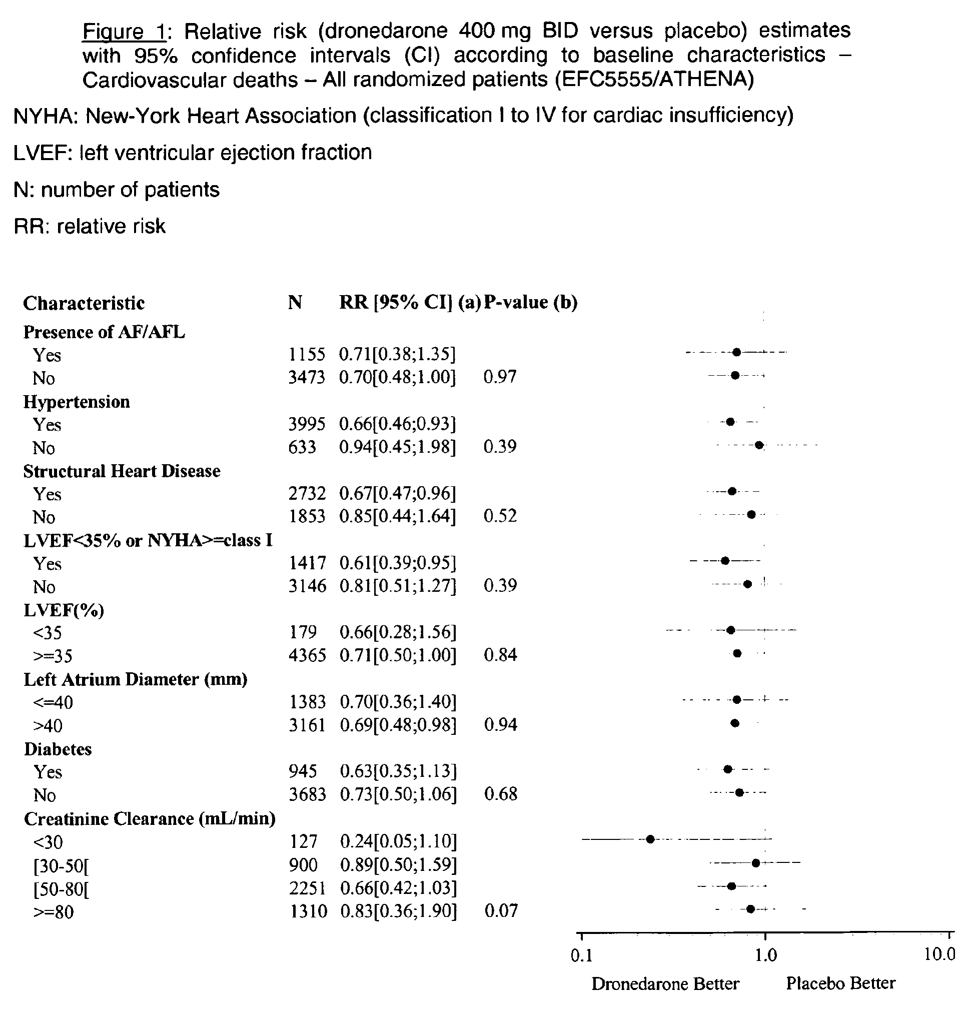

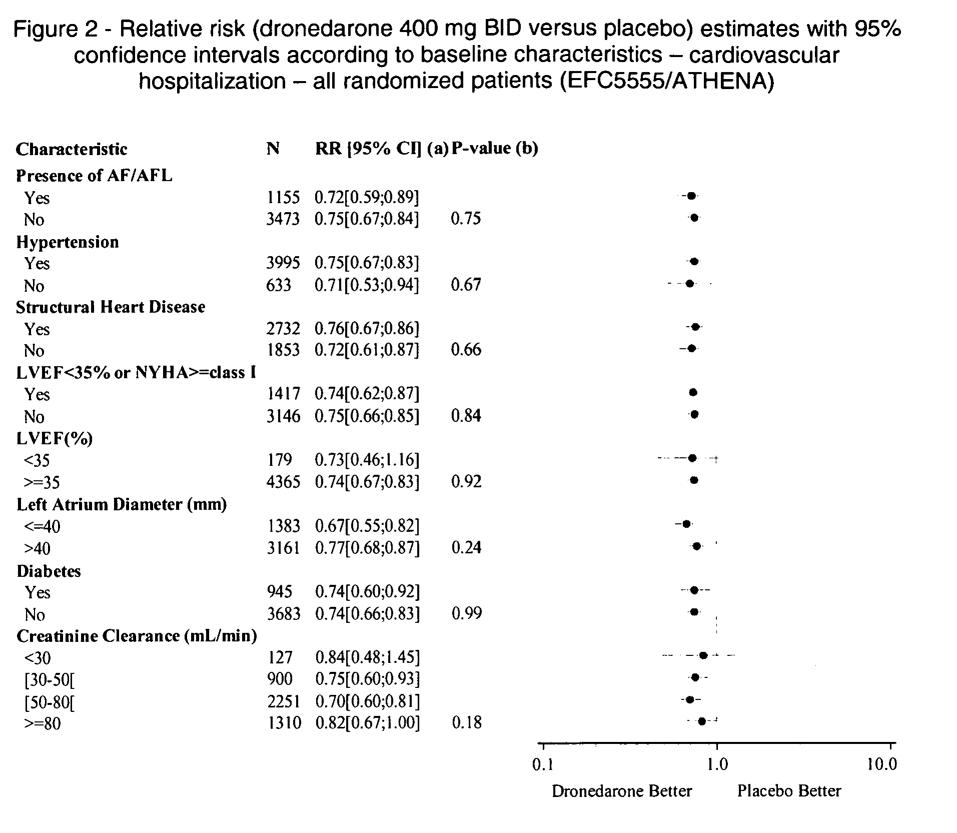

### Example 5

ACE/All have been largely used in ATHENA clinical study and at the same rate in the dronedarone group and in the placebo group. More than 75% of the patients had received one of these compounds during the study. Table 5 shows the numbers and percentages of patients using various medications at the inclusion in the study, whereas table 6 shows the numbers and percentages of patients who received concomitant medications during the study. Diuretics with potassium sparing properties, and among them spirolonolactone, have also been prescribed in the ATHENA trial.

**Table 5: Number (%) baseline selected medications - All randomized patients**

| | **Placebo (N=2327)** | **Dronedarone 400 mg BID (N=2301)** | **Total (N=4628)** |
|---|---|---|---|
| Beta blocking agents (except sotalol) | 1641 ( 70.5%) | 1628 ( 70.8%) | 3269 ( 70.6%) |
| **ACE inhibitors or A II receptor antagonists** | **1602 ( 68.8%)** | **1614 ( 70.1%)** | **3216 ( 69.5%)** |
| Oral anticoagulant | 1384 ( 59.5%) | 1403 ( 61.0%) | 2787 ( 60.2%) |
| Diuretics Diuretics other than spironolactone **Spironolactone** | 1265 ( 54.4%) 1224 ( 52.6%) **136 ( 5.8%)** | 1227 ( 53.3%) 1187 ( 51.6%) **148 ( 6.4%)** | 2492 ( 53.8%) 2411 ( 52.1%) **284 ( 6.1%)** |
| Low dose of aspirin (<= 365 mg) | 1019 ( 43.8%) | 1018 ( 44.2%) | 2037 ( 44.0%) |
| Statins Statins metabolized by CYP3A4 Statins not metabolized by CYP3A4 | 914 ( 39.3%) 755 ( 32.4%) 166 ( 7.1%) | 878 ( 38.2%) 737 ( 32.0%) 147 ( 6.4%) | 1792 ( 38.7%) 1492 ( 32.2%) 313 ( 6.8%) |
| Calcium antagonists with heart rate lowering effects | 307 ( 13.2%) | 331 ( 14.4%) | 638 ( 13.8%) |
| Digitalis | 308 ( 13.2%) | 321 ( 14.0%) | 629 ( 13.6%) |
| Drugs interacting with the creatinine tubular secretion | 237 ( 10.2%) | 229 ( 10.0%) | 466 ( 10.1%) |
| Moderate inhibitors of CYP3A4 | 226 ( 9.7%) | 214 ( 9.3%) | 440 ( 9.5%) |
| Other chronic antiplatelet therapy | 166(7.1%) | 126 ( 5.5%) | 292 ( 6.3%) |
| NSAID | 123 ( 5.3%) | 114 ( 5.0%) | 237 ( 5.1%) |

**Table 6: Number (%) of patients who received concomitant medications - All randomized patients**

| | **Placebo (N=2327)** | **Dronedarone 400 mg BID (N=2301)** |
|---|---|---|
| Beta blocking agents (except Sotalol) | 1860 ( 79.9%) | 1785 ( 77.6%) |
| **ACE inhibitors / A II receptor antagonists** | **1800 ( 77.4%)** | **1771 ( 77.0%)** |
| Oral anticoagulant | 1643 ( 70.6%) | 1601 ( 69.6%) |
| Diuretics Diuretics other than Spironolactone **Spironolactone** | 1559 ( 67.0%) 1522 ( 65.4%) **262 ( 11.3%)** | 1524 ( 66.2%) 1492 ( 64.8%) **257 ( 11.2%)** |
| Low dose of aspirin (<= 365 mg) | 1231 ( 52.9%) | 1225 ( 53.2%) |
| Statins Metabolized by CYP3A4 Not metabolized by CYP3A4 | 1131 ( 48.6%) 973 ( 41.8%) 305 ( 13.1%) | 1044 ( 45.4%) 909 ( 39.5%) 264 ( 11.5%) |
| Digitalis | 574 ( 24.7%) | 468 ( 20.3%) |
| Calcium antagonists with heart rate lowering effects | 490 ( 21.1%) | 459 ( 19.9%) |
| Drugs interacting with the creatinine tubular secretion | 434 ( 18.7%) | 382 ( 16.6%) |
| Moderate inhibitors of CYP3A4 | 369 ( 15.9%) | 323 ( 14.0%) |
| NSAID | 359 ( 15.4%) | 308 ( 13.4%) |
| Other chronic antiplatelet therapy | 260 ( 11.2%) | 182 ( 7.9%) |

## Claims

1. A method of managing the risk of an increased morbidity and mortality in patients treated by an association of dronedarone and of at least a compound B selected from ACE inhibitors, angiotensin II receptor antagonists and potassium sparing diuretics which method comprises the following steps:
a) initially measuring serum creatinine levels, which provides a reference level,
b) monitoring serum creatinine levels at regular intervals during treatment of the patient by dronedarone and by the compound B,
c) if the serum creatinine levels increases above the reference level, determining if the increase is due to dronedarone, to compound B or to another cause,
d) if the increase in the serum creatinine level above the reference level is due to dronedarone, then the treatment with dronedarone and compound B can be pursued; if the increase in the serum creatinine level above the reference level is due to compound B, then the treatment with compound B may be interrupted; if the increase in the serum creatinine level above the reference level is due to another cause than administration of dronedarone or of compound B, then appropriate treatment of such another cause shall be undertaken,
e) repeating steps b), c) and d) for the duration of the treatment with dronedarone and compound B.

2. A method of treatment of patients with cardiac arrhythmia comprising the administration of dronedarone to patients treated, in addition, by at least a compound B selected from ACE inhibitors, angiotensin II receptor antagonists and potassium sparing diuretics, which method comprises the steps a) to e) as defined in claim 1.

3. The method according to claim 2, for the treatment of patients with atrial fibrillation or atrial flutter.

4. The method according to any of claims 1 to 4, wherein the serum creatinine level in step a) is measured within 3 to 11 days after initiation of treatment with dronedarone.

5. The method according to claim 5, wherein the serum creatinine level in step
a) is measured within one week after initiation of treatment with dronedarone.

6. The method according to any of claims 1 to 5, wherein the monitoring of the serum creatinine level is effected according to the prescription labelling for the compound B.

7. The method according to any of claims 1 to 6, wherein step c) is achieved by temporarily interrupting treatment with dronedarone, while maintaining treatment with compound B.

8. The method according to claim 7, wherein the interruption of dronedarone treatment ranges from 1 to 2 weeks.
